# EUROPEAN PATENT APPLICATION

(11) **EP 2 080 508 A1**
(43) Date of publication of application: **22.07.2009**
(21) Application number: 08000629.9
(22) Date of filing: 15.01.2008
(51) Int. Cl.: A61K 9/00, A61K 9/14, A61K 31/137, A61K 31/439, C07D 453/02

(54) **Dry powder formulation comprising an anticholinergic drug**

(71) Applicant: CHIESI FARMACEUTICI S.p.A., I-43100 Parma (IT)
(72) Inventor: Brambilla, Gaetano, 43100 Parma (IT); Musa, Rosella, 43100 Parma (IT); Cocconi Daniela, 43100 Parma (IT)
(74) Representative: Minoja, Fabrizio

(57) **Abstract**

The invention relates to a pharmaceutical formulation in the form of inhalable dry powder comprising particles of a pharmaceutically acceptable salt of 3-[[[(3-fluorophenyl)[(3,4,5-trifluoro phenyl)methyl]amino] carbonyl]oxy]-1-[2-oxo-2-(2-thienyl)ethyl]-1-azoniabicyclo [2.2.2]octane as active ingredient, and particles of a carrier made of a physiologically acceptable pharmacologically-inert material.

The invention also relates to the process for the preparation thereof, and to its use in the prevention and/or treatment of a respiratory disease such as asthma and COPD.

## Description

### FIELD OF THE INVENTION

The invention relates to a dry powder formulation suitable to be administered by inhalation by means of a dry powder inhaler comprising a salt of 3-[[[(3-fluorophenyl)[(3,4,5-trifluorophenyl)methyl]amino] carbonyl]oxy]-1-[2-oxo-2-(2-thienyl)ethyl]-1-azoniabicyclo [2.2.2]octane as active ingredient.

The invention also relates to the process for the preparation thereof, and to its use in the prevention and/or treatment of an inflammatory or obstructive airways disease such as asthma and COPD.

### BACKGROUND TO THE INVENTION

Quaternary ammonium salts acting as muscarinic receptors antagonists are currently used in therapy to induce bronchodilation for the treatment of respiratory diseases, and in particular, inflammatory or obstructive airway diseases such as asthma and chronic obstructive pulmonary disease (COPD).

For treating chronic diseases, it is often desirable to utilize antimuscarinic drugs with a long-lasting effect. This ensures that the concentration of the active substance necessary for achieving the therapeutic effect is present in the lungs for a long period of time, without the need for the active substance to be administered repeatedly and too frequently.

In particular, it would be desirable to utilise antimuscarinic drugs which are therapeutically efficacious upon administration by inhalation once a day.

In order to fulfill such a requirement, antimuscarinic drugs shall exhibit good selectivity for M3 muscarinic receptors and slow dissociation from them.

Recently it has been reported that tiotropium bromide, the first drug in a new generation of antimuscarinic drugs, exhibits a very slow dissociation from M3 receptors, behaviour thought to account for its long lasting activity. However tiotropium bromide still retains a slow dissociation kinetics for the M2 muscarinic receptors Since M2 receptors are a major population in the cardiac muscle, a therapy with said drug might be accompanied by undesired cardiac side effects.

The quaternary ammonium salt 3-[[[(3-fluorophenyl)[(3,4,5-trifluoro phenyl)methyl]amino] carbonyl] oxy]-1-[2-oxo-2-(2-thienyl)ethyl]-1-azoniabicyclo[2.2.2]octane (hereinafter indicated as compound 1) is a novel compound which has been disclosed in the co-pending Patent Application no. PCT/EP2007/057585, incorporated herein by reference. The preparation of the (R) enantiomer of compound 1 is reported in Example 1 below.

The compound 1 has the following structure: 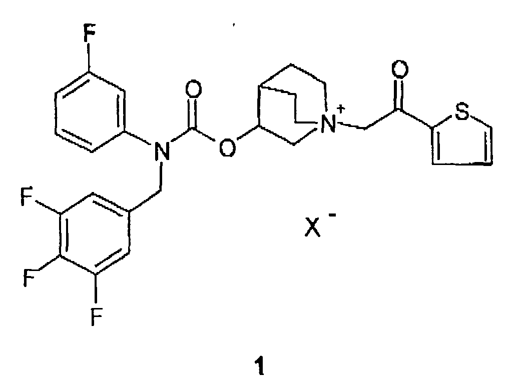 wherein X⁻ is a pharmaceutically acceptable anion preferably selected from the group consisting of chloride, bromide, iodide, sulfate, phosphate, methanesulfonate, nitrate, maleate, acetate, citrate, fumarate, tartrate, oxalate, succinate, benzoate and *p*-toluenesulfonate.

In particular the chloride salt of compound 1, has been found to be equieffective to tiotropium bromide in terms of receptor potency and duration of action, but significantly short-acting on the M2 receptors.

Therefore compound 1 may provide significant therapeutic benefit in the treatment of respiratory diseases such as asthma and COPD, when administered by inhalation.

Antimuscarinic drugs could be administered to the respiratory tract by inhalation in the form of dry powder by means of suitable inhalers known as dry powder inhalers (DPls).

The aim of the present invention is to provide an inhalable dry powder composition that comprise a pharmaceutically acceptable salt of 3-[[[(3-fluorophenyl)[(3,4,5-trifluoro phenyl)methyl]amino]carbonyl]oxy]-1-[2-oxo-2-(2-thienyl)ethyl]-1-azoniabicyclo [2.2.2]octane (compound 1) as active ingredient.

Optimally said formulation shall exhibit good flowability, good uniformity of distribution of the active ingredient and adequate chemical and physical stability in the device before use.

It shall also give rise to a good respirable fraction as well as deliver an accurate therapeutically active dose of the active ingredient.

### SUMMARY OF THE INVENTION

One aspect of the present invention provides a pharmaceutical formulation in the form of inhalable dry powder comprising micronised particles of a pharmaceutically acceptable salt of 3-[[[(3-fluorophenyl)[(3,4,5-trifluoro phenyl)methyl]amino] carbonyl]oxy]-1-[2-oxo-2-(2-thienyl)ethyl]-1-azoniabicyclo [2.2.2]octane (compound 1) as active ingredient and particles of a physiologically acceptable pharmacologically-inert solid carrier.

According to another aspect the present invention provides a dry powder inhaler comprising with said inhalable dry powder of the invention.

The present invention also relates to the use of the inhalable dry powder formulation described before as a medicament.

A further aspect of the present invention refers to the use of the inhalable dry powder described before for the prevention and/or treatment of an inflammatory or obstructive airways disease such as asthma or chronic obstructive pulmonary disease (COPD).

A still further aspect of the present invention refers to a method of preventing and/or treating an inflammatory or obstructive airways disease such as asthma or chronic obstructive pulmonary disease (COPD), which comprises administration by inhalation of an effective amount of the inhalable dry powder described before.

Finally the present invention is directed to a package comprising an inhalable dry powder formulation described before and a dry powder inhaler.

### DEFINITIONS

The terms "active drug", "active ingredient", "active" and "active substance", "active compound" and "therapeutic agent" are used as synonymous.

The terms "muscarinic receptor antagonists", antimuscarinic drugs" and "anticholinergic drugs" are used as synonymous.

As used herein, the term "substantially pure" means an active ingredient having an optical purity higher than 95% w/w, preferably higher than 98% w/w.

Full single therapeutically effective dose" means the quantity of active ingredient administered at one time by inhalation upon actuation of the inhaler.

Said full single dose may be delivered in one or more actuations, preferably one actuation (shot) of the inhaler.

"Actuation" means the release of active ingredient from the device by a single activation (e.g. mechanical or breath).

In general terms, the particle size of particles is quantified by measuring a characteristic equivalent sphere diameter, known as volume diameter, by laser diffraction.

The particle size can also be quantified by measuring the mass diameter by means of suitable instrument well known to the skilled person such as, for instance the sieve analyser.

The volume diameter (VD) is related to the mass diameter (MD) by the density of the particles (assuming a size independent density for the particles).

In the present application, the particle size is expressed in terms of mass diameter and the particle size distribution is expressed in terms of: i) the mass median diameter (MMD) which corresponds to the diameter of 50 percent by weight or volume respectively, of the particles, and ii) the MD in micron of 10% and 90% of the particles, respectively.

As used herein, the term "good flowability" refers to a formulation that is easy handled during the manufacturing process and is able of ensuring an accurate and reproducible delivering of the therapeutically effective dose.

Flow characteristics can be evaluated by measuring the Carr"s index; a Carr"s index of less than 25 is usually taken to indicate good flow characteristics.

As used herein, the expression "good homogeneity" refers to a formulation wherein, upon mixing, the content uniformity of the active ingredient, expressed as relative standard deviation (RSD), is less than 5%.

As used herein, the expression "chemically stable" refers to a formulation that meets the requirements of the ICH Guideline Q1A referring to "Stability Testing of new Active Substances (and Medicinal Products)".

As used herein, the expression "physically stable in the device before use" refers to a formulation wherein the active particles do not substantially segregate and/or detach from the surface of the carrier particles during fabrication of the dry powder and in the delivery device before use.

The tendency to segregate can be evaluated according to Staniforth et al. J. Pharm. Pharmacol. 34,700-706, 1982 and it is considered acceptable if the distribution of the active ingredient in the powder formulation after the test, expressed as relative standard deviation (RSD), does not change significantly with respect to that of the formulation before the test.

As used herein, the expression "respirable fraction" refers to an index of the percentage of active particles which would reach the deep lungs in a patient.

The respirable fraction, also termed fine particle fraction, is evaluated using a suitable *in vitro* apparata such as Multistage Cascade Impactor or Mutli Stage Liquid Impinger (MLSI) according to procedures reported in common Pharmacopeias.

It is calculated by the ratio between the respirable dose and the delivered dose.

The delivered dose is calculated from the cumulative deposition in the apparatus, while the respirable dose (fine particle dose) is calculated from the deposition on Stages 3 (S3) to filter (AF) corresponding to particles ≤ 4.7 microns.

A respirable fraction higher than 30% is an index of good inhalatory performances.

As used herein, the expression "accurate therapeutically active dose of the active ingredient" refers to a formulation wherein the variation between the mean delivered single dose and the mean emitted dose is equal to or less than 15%, preferably less than 10%.

### DETAILED DESCRIPTION OF THE INVENTION

The compositions of the invention are pharmaceutical formulations in the form of inhalable dry powder comprising micronised particles of a pharmaceutically acceptable salt of 3-[[[(3-fluorophenyl)[(3,4,5-trifluoro phenyl)methyl]amino] carbonyl]oxy]-1-[2-oxo-2-(2-thienyl)ethyl]-1-azoniabicyclo [2.2.2]octane (compound 1) as active ingredient, and particles of a of a physiologically acceptable pharmacologically-inert solid carrier (hereinfater the carrier).

The compound 1 has the following structure: 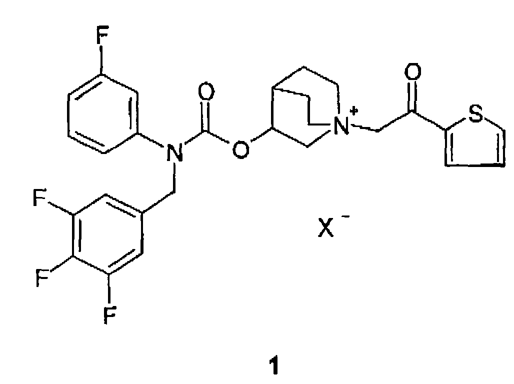 wherein the anion X⁻ is selected for the group consisting of chloride, bromide, iodide, sulfate, phosphate, methanesulfonate, nitrate, maleate, acetate, citrate, fumarate, tartrate, oxalate, succinate, benzoate and p-toluenesulfonate.

Compound 1 is preferably used in the form of chloride salt.

It will be apparent that compound 1 displays an asymmetric carbon on the quinuclidine ring and hence may be in the form of a mixture of two optical stereoisomers, (3R)- and (3S)-stereoisomers.

Compound 1 is preferably used in the form of substantially pure (3R)-enantiomer.

The (3R)-enantiomer of compound 1 in the form of chloride salt is hereinafter referred to as compound 1".

The compositions according to the invention comprise the active ingredient in an amount such that, in case of administration by inhalation from inhalers, the full single therapeutically effective dose (hereinafter the single dose) is advantageously comprised between about 1 µg and about 80 µg, preferably between about 5 µg and about 40 µg, expressed as compound 1".

Said dose will depend on the kind and the severity of the disease and the conditions (weight, sex, age) of the patient and shall be administered one or more times a day, preferably once a day.

In one embodiment, the single dose of a pharmaceutical composition comprising compound 1" is comprised between 2 µg and 10 µg. In other embodiments, the single dose is comprised between 10 µg and 15 µg or comprised between 15 µg and 20 µg.

In further embodiments the single dose is comprised between 20 µg and 40 µg.

In certain of the above embodiments the single dose is 10 µg, while in other ones is 20 µg or 40 µg.

The quantities of active substance in the compositions which are administered per single dose can be calculated analogously if another salt is used instead of the chloride salt of compound 1.

The particles of the salt of compound 1 in the formulation according to the invention must be in a finely divided (micronised) form, i.e. their mass median diameter should generally be equal to or less than 10 micron, preferably less than 6 micron, more preferably comprised between 1 and 6 micron.

The active ingredient may be produced in the desired particle size using known methods, e.g. milling, direct precipitation, spray-drying, freeze-drying or supercritical fluids.

The carrier particles may be made of any physiologically acceptable pharmacologically- inert material or combination of materials suitable for inhalatory use.

For example, the carrier particles may be composed of one or more materials selected from sugar alcohols; polyols, for example sorbitol, mannitol and xylitol and crystalline sugars, including monosaccharides and disaccharides; inorganic salts such as sodium chloride and calcium carbonate; organic salts such as sodium lactate; and other organic compounds such as urea, polysaccharides, for example starch and its derivatives; oligosaccharides, for example cyclodextrins and dextrins.

Advantageously, the carrier particles are made of a crystalline sugar, for example, a monosaccharide such as glucose or arabinose, or a disaccharide such as maltose, saccharose, dextrose or lactose.

Preferably, the carrier particles are made of lactose, more preferably of alpha-lactose monohydrate.

In one embodiment the invention the powder formulation may be in form of agglomerated spheronized particles, also known as soft pellets, wherein the particles of the salt of compound 1 and the particles of the carrier are both in a finely divided form, i.e. their mass median diameter is generally less than 10 micron, preferably from 1 to 6 micron.

Said kind of formulations may be prepared according to methods known to the skilled person.

Generally, the process comprises the steps of:
i) micronising together the active ingredient and the carrier;
ii) subjecting the resulting co-micronized mixture to agglomeration and spheronisation.

Alternatively, the process comprises the following steps:
i) micronising separately the active ingredient and the carrier;
ii) mixing the micronized components; and
iii) subjecting the resulting mixture to agglomeration and spheronisation.

In another embodiment of the invention, the formulation comprises coarse particles of a carrier together with the drug in the finely divided form, a type of formulation known in the art as ordered mixture.

Advantageously, said coarse carrier particles have a mass diameter (MD) of at least 50 micron, more advantageously greater that 90 micron. Preferably the MD is comprised between 50 micron and 500 micron.

In certain embodiments of the invention, the MD is comprised between 90 and 150 micron.

In other embodiments, the MD is comprised between 150 and 400 micron, and preferably between 210 and 355 micron.

When their MD is comprised between 150 and 400 micron, the coarse carrier particles have preferably a relatively highly fissured surface, that is, on which there are clefts and valleys and other recessed regions, referred to herein collectively as fissures.

The "relatively highly fissured" coarse particles can be defined in terms of fissure index or rugosity coefficient as described in WO 01/78695 and WO 01/78693, incorporated herein by reference, and they can be characterized according to the description therein reported.

Said coarse carrier particles may also be characterised in terms of tapped density or total intrusion volume measured as reported in WO 01/78695.

The tapped density of the coarse carrier particles is advantageously less than 0.8 g/cm³, preferably between 0.8 and 0.5 g/cm³.

The total intrusion volume is of at least 0.8 cm³ preferably at least 0.9 cm³.

When the formulation of the invention is in form of the aforementioned ordered mixture, it may advantageously comprise an additive material able to promote the release of the active particles from the carrier particles on actuation of the inhaler device, and hence able of improving the respirable fraction.

The additive material, which is preferably bound to the surface of the coarse carrier particles, is of a different material from the carrier particles.

Advantageously, the additive material is an amino acid, preferably selected from the group consisting of leucine, isoleucine, lysine, valine, methionine, phenylalanine. The additive may be a salt of a derivative of an amino acid, for example aspartame or acesulfame K.

In one embodiment of the invention the additive particles consist substantially of leucine, advantageously L-leucine.

Alternatively, the additive material may include or consist of one or more water soluble surface active materials, for example lecithin, in particular soya lecithin.

In a particular embodiment of the invention the additive material may include or consist of one or more lubricant selected from the group consisting of stearic acid and salts thereof such as magnesium stearate, sodium lauryl sulphate, sodium stearyl fumarate, stearyl alcohol, sucrose monopalmitate.

Other possible additive materials include talc, titanium dioxide, aluminium dioxide and silicon dioxide.

Advantageously, at least 90% by weight of the additive particles has a mass diameter (MD) of less than 35 micron. Advantageously, the MMD of the additive particles is not more than 25 micron, preferably not more than 15 micron, and more preferably not more than 10 micron.

The optimum amount of additive material shall depend on the chemical composition and other properties of the additive material.

In general, the amount of additive shall be not more than 10% by weight, based on the total weight of the formulation.

However, it is thought that for most additives the amount of additive material should be not more than 5%, preferably not more than 2% or even not more than 1% by weight or not more than 0.5% based on the total weight of the formulation. In general, the amount of additive material is of at least 0.01% by weight based on the total weight of the formulation.

In one of the preferred embodiment of the invention, the additive material is magnesium stearate.

The amount of magnesium stearate is generally comprised between 0.01 and 2%, preferably between 0.02 and 1%, more preferably between 0.1 % and 0.5% by weight based on the total weight of the formulation.

Magnesium stearate may cover the surface of the carrier particles in such a way as that the extent of the molecular surface coating is at least of 5%, preferably more than 10%, more preferably more than 15%, even more preferably equal to or more than and 25%.

The extent of molecular surface coating, which indicates the percentage of the total surface of the carrier particles coated by magnesium stearate, may be determined by water contact angle measurement as reported in WO 00/53157 or by electron scanning microscope.

The formulations of the invention in the form of ordered mixture may also comprise fine particles of a physiologically acceptable pharmacologically- inert material with a mass median diameter (MMD) equal to or less than 10 micron.

The percentage of fine particles of physiologically acceptable pharmacologically-inert material is advantageously comprised between 0.1 and 40% of the total amount of the formulation.

Preferably, the coarse particles and the fine particles are constituted of the same physiologically acceptable pharmacologically- inert material.

In a particularly preferred embodiment of the invention, a formulation according to the teaching of WO 01/78693 is provided, said formulation comprising:
i) particles of a salt of compound 1 in a micronised form
ii) a fraction of microparticles constituted of a mixture composed of particles of physiologically acceptable pharmacologically-inert material and particles of an additive material, said microparticles having a MMD equal to or less than 10 micron; and
iii) a fraction of particles of a physiologically acceptable pharmacologically-inert material having a mass diameter (MD) comprised between 150 micron and 400 micron, preferably between 212 and 355 micron.

Advantageously, the fraction of microparticles is composed of 90 to 99.5% by weight of the physiologically acceptable pharmacologically-inert material and 0.5 to 10% by weight of the additive material, and the ratio between the fraction of microparticles and the fraction of coarse particles is comprised between 1:99 and 40:60% by weight, preferably between 5:95 and 30:70% by weight, even more preferably between 10:90 and 20:80% by weight.

Preferably, the physiologically acceptable inert material is a-lactose monohydrate, and the additive material is magnesium stearate.

In a more preferred embodiment, the fraction of microparticles is composed of 98 to 99% by weight of α-lactose monohydrate and 1 to 2% by weight of magnesium stearate and the ratio between the fraction of microparticles and the fraction of coarse particles made of α-lactose monohydrate is 10:90% by weight, respectively.

The amount of magnesium stearate in the final formulation is advantageously comprised between 0.01 and 1.0% by weight, preferably between 0.05 and 0.5% by weight, more preferably between 0.1 and 0.4% by weight on the total weight of the formulation.

Magnesium stearate is added to said formulation with the aim of improving the respirable fraction of the active ingredient.

The formulation in form of ordered mixture according to the invention may be prepared according to well known methods.

Said methods comprise the step of mixing together the coarse carrier particles, the optional fine carrier particles and the additive particles and finally adding the finely divided pharmaceutically active compound to the resulting mixture.

The particularly preferred formulation according to the invention may be prepared according to the methods reported in WO 01/78693.

Among the methods therein described, the formulation is preferably prepared according to a process which comprises the following steps:
a) preparing microparticles constituted of a mixture composed of particles made of physiologically acceptable pharmacologically-inert material and particles of the additive, the inert material and the additive being first-mixed together and then co-micronised;
b) mixing the microparticles of step a) with coarse particles of a physiologically acceptable pharmacologically-inert material such that microparticles adhere to the surface of the coarse particles;
c) mixing the active particles in the micronized form with the particles of step b).

The co-micronization step may be carried out by known methods such as those reported in WO 02/00197.

Preferably said step is carrier out by milling, more preferably by using a jet mill according to the conditions reported in WO 01/78693.

Advantageously, during the step a) the additive may be embedded in the formed microparticles or, alternatively, in the case of a lubricant such as magnesium stearate, the additive may coat the surface of the carrier particles in such a way as that the extent of molecular surface coating is at least of 5%, preferably more than 10%, more preferably more than 15%, even more preferably more than 35%.

The extent of molecular surface coating indicates the percentage of the total surface of the carrier particles coated by magnesium stearate.

The presence of the additive material embedded in the microparticles may be detected according to known methods, for instance, by electron scanning microscope coupled to microcalorimetry.

On the contrary, as reported above, the extent of molecular surface coating may be determined by water contact angle measurement as reported in WO 00/53157 or by electron scanning microscope.

The formulations of the invention may further comprise other therapeutic agents useful for the prevention and/or treatment of a respiratory disease, e.g. corticosteroids such as budesonide and its epimers, beclometasone dipropionate, triamcinolone acetonide, fluticasone propionate, flunisolide, mometasone furoate, rofleponide and ciclesonide, anticholinergic or antimuscarinic agents such as ipratropium bromide, oxytropium bromide, tiotropium bromide, glycopyrrolate bromide and the group of phosphodiesterase-4 (PDE-4) inhibitors such as roflumilast, and their combinations.

The dry powder formulation herein described may be used in all customary dry powder inhalers.

Advantageously said formulation is filled in a multidose dry powder inhaler comprising a powder reservoir such as that described in WO 2044/012801.

Administration of the formulations of the invention may be indicated for prevention and/or the treatment of mild, moderate or severe acute or chronic symptoms or for prophylactic treatment of an inflammatory or obstructive airways disease such as asthma and chronic obstructive pulmonary disease (COPD).

Other respiratory disorders characterized by obstruction of the peripheral airways as a result of inflammation and presence of mucus such as chronic obstructive bronchiolitis and chronic bronchitis may also benefit from the formulation of the invention.

The invention is better illustrated by the following examples.

### EXAMPLES

### Example 1 - Preparation of (3R)-3-[(3-Fluorophenyl)-(3,4,5-trifluorobenzyl)carbamoyloxy]-1-(2-oxo-2-thlophen-2-yl-ethyl)-1-azoniabicyclo[2.2.2]octane chloride (compound 1')

A solution of (3R)-(3-fluorophenyl)-(3,4,5-trifluorobenzyl)carbamic acid 1-aza-bicyclo[2.2.2]oct-3-yl ester hydrochloride (prepared as described in WO03/053966 as Intermediate 15) (1 g, 2.248 mmol) in water (5 ml) was added with an excess of sodium carbonate (1 g) in water (4 ml) and extracted with ethyl acetate (2x10 ml). The organic layer was dried over anhydrous sodium sulfate and evaporated under vacuum.

The resulting free base (750 mg, 1.836 mmol) was dissolved in acetonitrile (4 ml) and added dropwise with a solution of 2-chloro-1-thiophen-2-yl-ethanone (750 mg, 3.657 mmol) in chloroform (6 ml). The resulting solution was refluxed for 12 hours. The solvent was evaporated and the residue dissolved in acetonitrile (7.5 ml). Crystallisation occurred after some minutes of stirring. The solid was filtered, washed with acetonitrile (1 ml) and dried under vacuum at 45°C. 760 mg of the title compound was obtained as white solid.

Mother liquors were evaporated to dryness and purified by column chromatography (SiO₂, eluent: dichloromethane/methanol =95/5 v/v to 80/20 v/v) to give further 120 mg of the title compound.

### Example 2 - Inhalable dry powder formulations comprising compound 1"

A powder formulation is prepared with the composition reported in **Table 1:**

**Table 1**

| *Components* | *Amounts* | | |
|---|---|---|---|
| | Per shot of the inhaler | | Single dose |
| | Mg | % | µg |
| Compound **1"** | 0.01 | 0.1 | 10 |
| alpha-lactose monohydrate 212-355 µm | 8.99 | 89.91 | |
| Pre-blend | 0.99 | 9.99 | |
| | | | |
| Total weight | 10 | | |

The final formulation is filled in the multidose dry powder inhaler described in WO 2004/012801.

The aerosol performances of said formulation are evaluated using a Multi Stage Liquid Impinger (MSLI) according to the procedure described in European Pharmacopoeia 2nd edition, 1995, part V.5.9.1, pages 15-17.

A further powder formulation is prepared with the composition reported in Table 2.

**Table 2**

| *Components* | *Amounts* | | |
|---|---|---|---|
| | Per shot of the inhaler | | Single dose |
| | mg | % | µg |
| Compound **1"** | 0.02 | 0.2 | 20 |
| alpha-lactose monohydrate 90-150 µm | 9.955 | 99.55 | |
| magnesium stearate | 0.025 | 0.25 | |
| | | | |
| Total weight | 10 | | |

### Example 3 - Assessment of the bronchodilation activity of compound 1"

Airway reactivity is measured using barometric plethysmography (Buxco, USA). Male guinea pigs (500-600 g) are individually placed in plexiglass chambers. After an acclimatisation period, animals are exposed to nebulised saline for 1 min to obtain airway baseline reading. This is followed by a 1 min challenge with nebulised acetylcholine (Ach) -2.5 mg/ml.

After 60 min, 5 min nebulisation of vehicle or the compound 1" in the range 2.5 - 250 mM are applied and Ach challenge is then repeated after 2, 5, 24, 48 and 72 h. Recording of pressure fluctuations in the chambers are taken for 5 min after each nebulisation and analysed to calculate Enhanced Pause (Penh). Airway reactivity is expressed as percentage increase in Penh compared with Penh values from the nebulisation of vehicle.

Two hours after the end of nebulisation with compound 1", the Ach-induced increase in Penh is dose-dependently inhibited by the compound, with a maximal effect of 99.2 ± 0.5 at 250 mM.

As for the time-course of the effect, compound 1" shows increasing duration of action with increasing dose.

After inhalation of 250 mM of compound **1"**, effect persists unchanged up to 48 h (83.0 ± 16.1 %), while at 72 h a residual activity of 34.8 ± 20.9% is present. Twenty-four hours after 25 and 50 mM compound 1" inhalation, a significant bronchoprotective effect was observed (87.1 ± 8.7% and 63.1 ± 19.2%, respectively). At 50 mM, a significant inhibition persists up to 48 h (49.2 ± 23.2%). Inhalation of lower concentrations results in an effect that did not exceed the 5 h observation point.

The estimation of lung levels of compound 1" achieved after nebulisation endowed with a submaximal bronchodilator activity at 2 h after treatment reveals that the its retained dose in the target organ is about 50 µg/kg. If an extrapolation of these results from rat to human is made, it can be predicted that in patients the therapeutically effective dose might be comprised between 1 and 80 µg, preferably between 5 and 40 µg, after administration by inhalation.

## Claims

1. An inhalable dry powder formulation comprising particles of a pharmaceutically acceptable salt of 3-[[[(3-fluorophenyl)[(3,4,5-trifluoro phenyl)methyl]amino] carbonyl]oxy]-1-[2-oxo-2-(2-thienyl)ethyl]-1-azoniabicyclo [2.2.2]octane (compound **1**) and particles of a carrier made of a physiologically acceptable pharmacologically-inert material.

2. The inhalable powder according to claim 1 wherein said salt is selected form the group consisting of chloride, bromide, iodide, sulfate, phosphate, methanesulfonate, nitrate, maleate, acetate, citrate, fumarate, tartrate, oxalate, succinate, benzoate and p-toluenesulfonate.

3. The inhlable powder according to claim 1 wherein said salt is the chloride salt of the (3R)-enantiomer (compound 1").

4. The inhalable powder according to claim 3 wherein compound 1" is administered at a full single therapeutically effective dose comprised between about 1 µg and about 80 µg.

5. The inhalable powder according to claim 4 wherein the single dose is comprised between about 5 µg and about 40 µg.

6. The inhalable powder according to any one of claims 1 to 5 wherein the carrier comprises a crystalline sugar selected from the group consisting of glucose, arabinose, maltose, saccharose, dextrose and lactose or a polyalcohol selected from the group consisting of mannitol, maltitol, lactitol and sorbitol.

7. The inhalable powder according to claim 6 wherein the sugar is lactose.

8. The inhalable powder according to claim 7 wherein the sugar is α-lactose monohydrate.

9. The inhalable powder according to any one of claims 1 to 8 wherein the carrier is in form of finely divided particles having a mass median diameter (MMD) equal to or of less than 10 micron.

10. The inhalable powder according to any one of the claims 1 to 8 wherein the carrier is in form of coarse particles having a mass diameter of at least 50 micron.

11. The inhalable powder according to claim 10 wherein the mass diameter is greater than 90 micron.

12. The inhalable powder according to claim 11 wherein the mass diameter is comprised between 150 and 400 micron.

13. The inhalable powder according to any one of the claims 1 to 8 wherein the carrier comprises a mixture of coarse particles having a mass diameter greater than 90 micron and finely divided particles with a MMD equal to or less than 10 micron.

14. The inhalable powder according to any one of claims 10 to 13 further comprising one or more additive materials selected form the group consisting of amino acids, water soluble surface active agents, lubricants and glidants.

15. The inhalable powder according to claim 14 wherein the additive material is leucine.

16. The inhalable powder according to claim 14 wherein the additive material is magnesium stearate.

17. The inhalable powder according to claim 16 wherein magnesium stearate is present in an amount comprised 0.01 and 2% by weight based on the total weight of the formulation.

18. The inhalable powder according to claim 17 wherein the amount of magnesium stearate is comprised between 0.02 and 1% w/w.

19. The inhalable powder according to claim 13 comprising:
i) particles of compound **1** in a finely divided form;
ii) a fraction of microparticles consisting of a mixture composed of particles of a physiologically acceptable pharmacologically-inert material and particles of an additive material, said microparticles having a MMD equal to or less than 10 micron; and
iii) a fraction of coarse particles of a physiologically acceptable pharmacologically-inert material having a mass diameter comprised between 150 and 400 micron.

20. The inhalable powder according to claim 19 wherein the mass diameter of the coarse particles is comprised between 212 and 355 micron.

21. The inhalable powder according to claim 20 wherein the fraction of microparticles is composed of 90 to 99.5% by weight of the physiologically acceptable pharmacologically inert material and 0.5 to 10% by weight of the additive material.

22. The inhalable powder according to claim 20 or 21 wherein the ratio between the fraction of microparticles and the fraction of coarse particles is comprised between 1:99 and 40:60% by weight.

23. The inhalable powder according to claim 22 wherein the ratio is comprised between 5:95 and 30:70% by weight.

24. The inhalable powder according to claim 23 wherein the ratio is comprised between 10:90 and 20:80% by weight.

25. The inhalable powder according to any one of claims 19 to 24 wherein the physiologically acceptable inert material is α-lactose monohydrate.

26. The inhalable powder according to any one of claims 19 to 25 wherein the additive material is magnesium stearate.

27. The inhalable powder according to any one of claims 22 to 26 wherein the fraction of microparticles is composed of 98 to 99% by weight of α-lactose monohydrate and 1 to 2% by weight of magnesium stearate and the ratio between the fraction of microparticles and the fraction of coarse particles made of α-lactose monohydrate particles is 10:90% by weight.

28. A dry powder inhaler comprising an inhalable dry powder formulation according to any one of claims 1 to 27.

29. Use of an inhalable dry powder formulation according to any one of claims 1 to 27 as a medicament.

30. Use of the inhalable dry powder formulation according to any one of claims 1 to 27 for the prevention and/or treatment of a respiratory disease.

31. The use according to claim 30 wherein the disease is asthma.

32. The use according to claim 30 wherein the disease is chronic obstructive pulmonary disease (COPD).

33. A package comprising an inhalable dry powder formulation according to any one of claims 1 to 27 and a dry powder inhaler.
